# EUROPÄISCHE PATENTSCHRIFT

(11) **EP 2 848 384 B1**
(45) Veröffentlichungstag und Bekanntmachung des Hinweises auf die Patenterteilung: **09.11.2016**
(21) Anmeldenummer: 14184626.1
(22) Anmeldetag: 12.09.2014
(51) Int. Cl.: B29C 49/46

(54) **Vorrichtung zum Umformen von Kunststoffvorformlingen mit Reinraum**
Device for reforming plastic pre-forms with a clean room
Dispositif de transformation de préformes en matière synthétique avec salle blanche

(30) Priorität: 12.09.2013 DE 202013008055 U
(43) Veröffentlichungstag der Anmeldung: 18.03.2015
(73) Patentinhaber: Krones AG, 93073 Neutraubling (DE)
(72) Erfinder: Winzinger, Frank, 93073 Neutraubling (DE); Geltinger, Florian, 93073 Neutraubling (DE)
(74) Vertreter: Bittner, Bernhard

(56) Entgegenhaltungen:
- EP-A1- 2 495 089
- EP-A1- 2 495 090
- EP-A1- 2 511 070
- EP-A2- 2 388 127
- EP-A2- 2 570 251
- WO-A1-2012/153268
- CN-A- 102 773 998

## Beschreibung

Die vorliegende Erfindung bezieht sich auf eine Vorrichtung zum Umformen von Kunststoffbehältnissen. Im Bereich der getränkeherstellenden Industrie ist es bekannt, Kunststoffbehältnisse zu verwenden, wobei diese Kunststoffbehältnisse durch einen Umformungsvorgang, und insbesondere einen Blasvorgang, aus Kunststoffvorformlingen erzeugt werden. Dabei sind üblicherweise Blasräder vorgesehen, an denen mehrere Blasstationen angeordnet sind, wobei innerhalb dieser Blasstationen die Kunststoffvorformlinge durch Beaufschlagung mit Druckluft gegen eine Innenwandung der entsprechenden Blasform expandiert werden. Derartige Blasräder sind bevorzugt als Rotor ausgebildet und drehen sich kontinuierlich um ihre Drehachse. Die Drehachse ist insbesondere vertikal ausgerichtet. Eine Verlängerung der Drehachse schneidet also den Erdmittelpunkt.

Für viele Getränke ist es dabei erforderlich, diese unter aseptischen Bedingungen abzufüllen. Dabei ist es bekannt, dass ein Sterilisationsprozess für das aseptische Abfüllen mit der Sterilisation der bereits fertig gestellten Flaschen in einem dafür vorgesehen Reinraum beginnt. Alle im Stand der Technik davor stattfindenden Prozesse wie die Preform-Herstellung, deren Transport, deren Erwärmung und deren Blasen zur Flasche finden in einer unsterilen Umgebung statt. In diesem Falle ist es erforderlich, eine relativ große Fläche, nämlich diejenige der fertig erzeugten Kunststoffflasche, zu sterilisieren.

Das Sterilisieren von Behältnissen, welche mit sensiblem Füllgut befüllt werden sollen, kann beispielsweise dadurch geschehen, dass das Füllgut erhitzt und heiß abgefüllt wird und mit dem heißen Füllgut die Flascheninnenseite mit entkeimt wird. Weiterhin ist es auch möglich, dass das leere Behältnis vor dem Füllvorgang separat entkeimt wird und unter aseptischen Bedingungen mit an anderer Stelle sterilisiertem Füllgut befüllt wird. Die Sterilität des leeren Behältnisses wird dabei durch chemische Desinfektionsmittel wie Peressigsäure (nass) oder Wasserstoffperoxid (trocken) erreicht. Dazu werden die Behältnisse in einen sogenannten Isolator gefahren, in dem sie mit dem Desinfektionsmittel beaufschlagt werden, welches eine bestimmte Zeit einwirken muss und anschließend mit hohem Aufwand wieder entfernt werden muss. Hierbei entsteht eine Restmengenproblematik. Direkt im Anschluss an diesen Isolator ist der aseptisch gekapselte Füller angeordnet. Diese Technologie ist jedoch noch relativ aufwändig.

Daher ist es meist wünschenswert, nicht die Kunststoffflasche selbst zu sterilisieren, sondern den Kunststoffvorformling, da dieser eine erheblich geringere Oberfläche aufweist. Allerdings ist es erforderlich, die Behältnisse nach deren Sterilisation, insbesondere durchgängig, unter sterilen Bedingungen zu transportieren, zumindest bis diese verschlossen sind, um auf diese Weise eine weitere Verunreinigung der Behältnisse zu verhindern.

Die EP 2 388 129 A1 weist eine Vorrichtung zum Umformen von Kunststoffvorformlingen zu Kunststoffbehältnissen mit einem Reinraum auf, in welchem die Blasformen, d. h. die Seitenteile und das Bodenteil angeordnet sind. Die Antriebseinrichtungen zum Bewegen der Seitenteile bzw. des Bodenteils sind jedoch wenigstens teilweise außerhalb des Reinraums angeordnet. Weiterhin sind Kopplungseinrichtungen zum Koppeln der Antriebseinrichtungen mit den Seitenteilen bzw. dem Bodenteil vorhanden, welche durch die Grenze des Reinraums hindurchtreten. Die Kopplungseinrichtungen sind daher vorteilhaft mit Faltenbälgen eingehaust, die eine translatorische bzw. rotatorische Bewegung der Kopplungseinrichtung ermöglichen, wobei gleichzeitig der Reinraum steril gehalten werden kann. Nachteilig bei der Verwendung von Faltenbälgen ist, dass Faltenbälge als Abdichtungsmittel sehr teuer sind und es zudem sehr schwierig ist, die ziehharmonikaartigen Zwischenräume der Faltenbalge zu reinigen bzw. sterilisieren.

Zur Lagerung der Formträgerhälften sind Schwenkachsen vorhanden, welche unbeweglich mit der oberen sowie der unteren Wandung verbunden sind. An der Stelle, an der die Schwenkachse die Reinraumgrenze durchtritt muss eine Dichtung, beispielsweise ein O-Ring, vorgesehen sein. Die Schwenkbewegung der Formträgerhälften um die Schwenkachsen wird dabei durch horizontal angebrachte Arme initiiert, welche eine Kopplungseinrichtung darstellen und mittels Faltenbälgen abgedichtet sind.

Die EP 2 495 086 A1 zeigt weiterhin Schwenkwellen, die zum Auseinanderklappen der Blasformträger dienen. Im Unterschied zur EP 2 388 129 A1 sind die Schwenkwellen drehbar an der oberen bzw. unteren Wandungen angebracht und es sind keine zusätzlichen Arme zur Verschwenkung notwendig. Eine Lagerung dieser Wellen kann sich außerhalb des Reinraums befinden. Details zur Lagerung sind dieser Anmeldung nicht zu entnehmen, da die Schwenkwellen aber jeweils mit Antriebseinrichtungen gekoppelt sind, ist naheliegend, dass die Schwenkwellen im Bereich der Antriebseinrichtungen gelagert sind und an der Grenze zum Reinraum durch die oben beschriebenen Faltenbälge abgedichtet werden. Problematisch an einer derartigen Lagerung ist, dass durch die Bewegung der Seitenteile an der Schwenkwelle Momente entstehen, die Kopplungseinrichtung stark belasten und zudem die Faltenbalge schwer reinig- bzw. sterilisierbar sind.

Weitere Vorrichtungen werden in EP2570251, EP2511070, EP2495090, CN102773998, EP2388127 und EP2495089 offenbart.

Der vorliegenden Erfindung liegt daher die Aufgabe zugrunde, die Lagerung einer Kopplungseinrichtung zu optimieren und dabei gleichzeitig den Aufwand zum Sterilisieren bzw. Sterilhalten einer Umformungseinheit, bzw. Blaseinrichtung für Behältnisse zu reduzieren. Dies wird erfindungsgemäß durch eine Vorrichtung nach Anspruch 1 erreicht. Vorteilhafte Ausführungsformen und Weiterbildungen sind Gegenstand der Unteransprüche.

Eine erfindungsgemäße Vorrichtung zum Umformen von Kunststoffvorformlingen zu Kunststoffbehältnissen weist daher eine Transporteinrichtung auf, an der eine Vielzahl von Blasstationen angeordnet ist, wobei jede dieser Blasstation eine Blasform aufweist und die Vorrichtung einen Reinraum aufweist, innerhalb dessen die Kunststoffvorformlinge transportiert werden können. Weiterhin weist jede Blasform zwei Seitenteile und ein Bodenteil auf, wobei die Seitenteile und das Bodenteil gemeinsam einen Hohlraum ausbilden, innerhalb dessen ein Kunststoffvorformling zu einem Kunststoffbehältnis umformbar ist. Dabei sind wenigstens die diesen Hohlraum begrenzenden Bereiche der Seitenteile und des Bodenteils stets innerhalb des Reinraums angeordnet. Weiterhin ist wenigstens eine Antriebseinrichtung zum Bewegen der Seitenteile oder wenigstens zum Bewegen des Bodenteils vorgesehen.

Dabei ist wenigstens eine der Antriebseinrichtungen bzw. die Antriebsrichtung wenigstens teilweise außerhalb des Reinraums angeordnet und eine Dichtungseinrichtung vorgesehen, welche den Reinraum gegenüber einem Bereich, in dem die Antriebseinrichtung angeordnet ist, abdichtet. Weiterhin ist wenigstens eine der außerhalb des Reinraums angeordneten Antriebseinrichtungen über eine Kopplungseinrichtung mit einem Seitenteil gekoppelt und diese Kopplungseinrichtung erstreckt sich durch zumindest eine den Reinraum begrenzende Wandung hindurch erstreckt.

Es wird erfindungsgemäß vorgeschlagen, eine Lagerungseinrichtung zum Lagern der Kopplungseinrichtung vorzusehen, die an der Wandung befestigt ist und sich von der Befestigung nur zu einer Seite der Wandung erstreckt.

Die erfindungsgemäße Vorrichtung hat den Vorteil, dass kein Faltenbalg zur Abdichtung benötigt wird, sondern lediglich eine kreisförmige Dichtung in Form eines Rings vorhanden sein muss. Somit lässt sich die Vorrichtung wesentlich besser reinigen bzw. sterilisieren.

Unter "nur zu einer Seite der Wandung erstreckt" versteht man, dass ein Ende der Lagerungseinrichtung an der Wandung befestigt ist und das andere Ende entweder in Richtung des Reinraums oder in Richtung der Umgebung zeigt. Die Lagerungseinrichtung erstreckt sich jedoch nicht durch die Wandung hindurch in beide Richtungen. Das erste Ende liegt immer an einem Außenbereich, an einem Innenbereich oder an einem Seitenbereich der Wandung und geht nur in einer Richtung über den Bereich der Wandung hinaus.

Der Innenbereich der Wandung ist die Oberfläche der Wandung, welche dem Reinraum zugewandt ist und welche sich in der Nähe des Durchtrittslochs der Kopplungseinrichtung befindet. Der Außenbereich ist die Oberfläche der Wandung, welche dem Reinraum abgewandt ist bzw. der Umgebung zugewandt ist und welche sich in der Nähe des Durchtrittslochs der Kopplungseinrichtung befindet. Der Seitenbereich ist die Oberfläche der inneren Seite des Durchtrittslochs der Kopplungseinrichtung durch die Wandung, welche sich parallel zur Achse der Kopplungseinrichtung erstreckt.

Eine Lagerungseinrichtung, die sich von der Wandung in den Reinraum erstreckt, hat den Vorteil, dass sich die Lagerungseinrichtung möglichst nahe an dem Punkt befindet, an dem die durch die Bewegung der Seitenteile entstehende Kraft auf die Kopplungseinrichtung einwirkt und diese Kraft dadurch besser abgefangen werden kann, um die Kopplungseinrichtung möglichst wenig zu verformen (Genauigkeit) bzw. zu belasten (Dauerfestigkeit).

Wenn sich die Lagerungseinrichtung allerdings von der Wandung in Richtung Umgebung erstreckt, ergibt sich der Vorteil, dass innerhalb des Reinraums weniger Flächen sind, an denen sich Keime ablagern können bzw. die sterilisiert werden müssen. Der Reinraum kann weiterhin kompakter, also insgesamt kleiner, ausgebildet werden.

Bevorzugt beginnt die Lagerungseinrichtung vollständig auf Höhe der Wandung und ist im Wesentlichen senkrecht zur Wandung angebracht.
Die Lagerungseinrichtung ist dabei vorzugsweise rohrförmig bzw. wie ein Hohlzylinder ausgebildet.
Unter einem "Beginnen vollständig auf Höhe der Wandung" versteht man, dass das eine Ende der Lagerungseinrichtung an dem Seiten-, Innen- oder Außenbereich der Wandung befestigt, bevorzugt verschweißt oder verschraubt, ist. Vollständig heißt dabei, dass sich das erste Ende der Lagerungseinrichtung, sofern das andere Ende in den Reinraum hineinragt, höchstens bis zur Ebene des Außenbereichs der Wandung erstreckt, aber nicht darüber hinaus geht. Das erste Ende kann demnach am Seitenbereich und/oder am Innenbereich, aber nicht am Außenbereich befestigt sein. Das gleiche gilt analog für eine Lagerungseinrichtung, die sich in Richtung Umgebung erstreckt. Diese endet dann allerdings in der Ebene des Innenbereichs und ist am Seitenbereich und / oder am Außenbereich angebracht.

Bei einer vorteilhaften Ausführungsform sind zwei Lagerungseinrichtungen zum Lagern der Kopplungseinrichtung vorhanden, wobei sich beide Lagerungseinrichtungen von der Wandung in Richtung Reinraum oder in Richtung Umgebung erstrecken. Insbesondere sind diese zwei Lagerungseinrichtungen an unterschiedlichen Wandungen angebracht, beispielsweise eine Lagerungseinrichtung an einer oberen Wand, die andere an einer unteren Wand des Reinraums.

Lagerungseinrichtungen, die sich in Richtung des Reinraums erstrecken, haben den Vorteil, dass die Lagerungseinrichtugen möglichst nah an der Stelle, an der durch die Bewegung der Seitenteile eine Kraft auf die Kopplungseinrichtung einwirkt, angreifen können und somit die Kopplungseinrichtungen weniger stark belastet werden. Vorteilhaft ist weiterhin, dass, wenn die gesamte Blasstation inklusive eines Teils der unteren Wandung ausgewechselt wird, das Modul der Blasstation auf einem ebenen Träger, insbesondere bodennah, oder direkt auf dem Boden abgestellt werden kann, da die Wandung von unten eine ebene Fläche darstellt.

Sofern sich die Lagerungseinrichtungen in Richtung der Umgebung erstrecken, ergibt sich der Vorteil, dass weniger zu sterilisierende Flächen im Reinraum angebracht sind, an welchen sich Keime anlagern können und der Reinraum somit leichter zu sterilisieren ist. Ein weiterer Vorteil ist, dass der ringförmige Reinraum möglichst kompakt ausgebildet werden kann.

Je nachdem, welche Anforderungen an die Vorrichtung im konkreten Fall bestehen, kann man die jeweilige Ausführungsform einsetzen.

Vorteilhaft sind zwei Lagerungseinrichtungen zum Lagern der Kopplungseinrichtung vorhanden, wobei sich die erste Lagerungseinrichtung von der Wandung in Richtung Reinraum und die zweite Lagerungseinrichtung in Richtung Umgebung erstreckt.

Insbesondere ist auch daran gedacht, die untere Lagerungseinrichtung im Reinraum anzuordnen und die obere außerhalb.

Bevorzugt ist die Lagerungseinrichtung unbeweglich mit der Wandung verbunden. Besonders bevorzugt ist die Lagerungseinrichtung im Betrieb unbeweglich mit der Wandung verbunden. Dabei kann die Lagerungseinrichtung entweder mit der Wandung verschweißt oder verschraubt oder verklemmt werden. Sofern die Lagerungseinrichtung mit der Wandung verschraubt ist, kann sie bei Stillstand der Maschine eingestellt werden.
Die Lagerungseinrichtung weist insbesondere zudem ein oder mehrere Lagerungsmittel auf, die eine Rotationsbewegung der Kopplungseinrichtung ermöglichen. Zudem ist zwischen Reinraum und Lagerungsmittel eine Dichtungseinrichtung angebracht. Diese Dichtungseinrichtung kann an der Lagerungseinrichtung angebracht sein und zwar derart, dass sie die Kopplungseinrichtung und die Lagerungseinrichtung berührt.
Alternativ ist es auch denkbar, zum Abdichten die gesamte Lagerungseinrichtung innerhalb eines Faltenbalgs anzubringen, welcher auf einer Seite an der Wandung und auf der anderen Seite an der Kopplungseinrichtung befestigt ist. Dabei ist keine weitere Dichtungseinrichtung zwischen Lagerungseinrichtung und Kopplungseinrichtung notwendig, da die gesamte Lagerungseinrichtung nicht mit dem Reinraum in Berührung kommt.
Die erste Variante zur Abdichtung ist jedoch hinsichtlich der Reinigungs- bzw. Sterilisationsfähigkeit sinnvoller, da die Lagerungseinrichtung bevorzugt eine glatte Oberfläche aufweist und somit wesentlich besser sterilisiert werden kann als ein ziehharmonikaartiger Faltenbalg. Vorteilhaft ist eine erste Lagerungseinrichtung mit der Wandung verschraubt und eine zweite Lagerungseinrichtung mit der Wandung verschweißt.
Es ist aber genauso denkbar, die erste und die zweite Lagerungseinrichtung mit der Wandung zu verschrauben oder beide mit der Wandung zu verschweißen

Die erste und die zweite Lagerungseinrichtung dienen dabei vorteilhafterweise zur Lagerung einer gemeinsamen Kopplungseinrichtung.
Die erste Lagerungseinrichtung kann beispielsweise an der oberen Wandung, also dem Deckel des Reinraums angebracht sein und die zweite Lagerungseinrichtung an der unteren Wandung, also dem Boden des Reinraums. Die Kopplungseinrichtung wird demnach beim Eintritt in den Reinraum und beim Austritt aus dem Reinraum durch jeweils eine Lagerungseinrichtung gelagert.

Vorteilhafterweise ist eine Fläche der Lagerungseinrichtung in einem Winkel zur Horizontalen ausgebildet, bevorzugt in einem Winkel von 5°-85°, besonders bevorzugt in einem Winkel von 10°-30°. Mit der Fläche ist insbesondere die Außenfläche der Lagerungseinrichtung innerhalb des Reinraums gemeint.
Dies hat den Vorteil, dass keine Flüssigkeiten auf horizontalen Flächen stehen bleiben können, sondern Flüssigkeiten immer in Richtung der unteren Wandung des Reinraums abfließen. Unter Flüssigkeiten ist unter anderem Kondensat, Reinigungsmedium wie Schaum oder Sterilwasser zur Beseitigung des Schaums zu verstehen.

Bevorzugt erstreckt sich die Lagerungseinrichtung zum Lagern der Kopplungseinrichtung von der Wandung in Richtung Reinraum und es ist weiterhin eine Abstützeinrichtung vorgesehen, die sich auf dem Außenbereich dieser Wandung befindet.
Die Abstützeinrichtung ist ebenfalls zum Führen bzw. Lagern der Kopplungseinrichtung geeignet. Die Abstützeinrichtung kann rohrförmig oder eckig ausgeführt sein.

Besonders bevorzugt ist die Abstützeinrichtung an eine Ausnehmung der Wandung zum Einsetzen bzw. Austauschen der gesamten Blasstation angepasst.

Vorteilhaft ist zwischen der Kopplungseinrichtung und der Lagerungseinrichtung eine Dichtungseinrichtung vorgesehen.

Diese Dichtungseinrichtung dichtet den Reinraum gegenüber der Umgebung ab, ermöglicht aber gleichzeitig eine Rotationsbewegung der Kopplungseinrichtung.
Bei der Dichtung kann es sich also um eine Rotationsdichtung handeln, beispielsweise um eine Lippendichtung.
Alternativ kann als Dichtungseinrichtung auch ein Faltenbalg verwendet werden, welcher die komplette Lagerungseinrichtung gegenüber dem Reinraum abschirmt.
In einer bevorzugten Ausführungsform kann es sich bei der Dichtungseinrichtung auch um eine hydraulische Dichtung, beispielsweise um ein Wasserschloss handeln.

Bevorzugt dient die als Hauptwelle ausgeführte Kopplungseinrichtung als Schwenkantrieb für ein Seitenteil der Blasform und das zweite Seitenteil wird über einen Kopplungsmechansimus zusammen mit der Verriegelung geschwenkt. Somit ist es möglich, dass die beiden Seitenteile zwar um die Hauptwelle geschwenkt werden, jedoch unterschiedlich weit geöffnet werden.

Der eigentliche Antrieb für die Schwenkbewegung kann beispielsweise mittels Kurvenrollen oder auch mittels Motoren an Hebeln angreifen, welche an der Schwenkwelle angeordnet sind.

Bevorzugt ist die Kopplungseinrichtung zusätzlich zur Betätigung eines Seitenteils zur Betätigung der Beaufschlagungseinrichtung, des Bodenteils und/oder der Verriegelung vorgesehen.

Die Verriegelung kann beispielweise an einer am Blasrad unbeweglich befestigten Formträgerhälfte angebracht sein, wobei lediglich die zweite Blasformhälfte schwenkbar angeordnet ist, oder die Verriegelung kann alternativ in einem Abstand zu den Formträgerhälften an der Wandung des Reinraums angebracht sein, so dass die Kopplungseinrichtung, also die sogenannte Verriegelungswelle, beispielsweise lediglich eine Drehbewegung um ihre Mittelachse ausführt. Sofern die Verriegelung lediglich eine Drehbewegung um die Mittelachse durchführen muss, kann die Lagerung und Abdichtung der Verriegelungswelle analog zur Lagerung und Abdichtung der Hauptwelle durchgeführt werden.
Es ist jedoch auch denkbar, dass die Verriegelungswelle eine mit der Öffnung der Blasform gekoppelte Bewegung senkrecht zur Drehachse der Hauptwelle durchführt.

Wenn auch noch eine derartige Schwenkbewegung vorgesehen ist, muss die Verriegelungswelle von einem Faltenbalg oder einer anderen Dichtung, die eine translatorische sowie eine rotatorische Bewegung ermöglicht, abgedichtet werden.

Die Beaufschlagungseinrichtung und das Bodenteil führen im Gegensatz zur Hauptwelle eine translatorische Bewegung im Wesentlichen senkrecht zur Fläche der oberen bzw. unteren Wandung durch. Auch bei einer solchen Bewegung kann die Lagerung der jeweiligen Kopplungseinrichtung durch eine an der Wandung befestigte Lagerungseinrichtung geschehen. Dabei muss jedoch darauf geachtet werden, dass die Kopplungseinrichtung keine Keime von der Umgebung in den Reinraum schleppt. Eine Verunreinigung des Reinraums kann beispielsweise umgangen werden, indem der Reinraum zumindest derart vergrößert wird, dass sich zumindest die Kopplungseinrichtung in einem rohrartigen Gehäuse befindet, in das keine Keime eindringen können. Das Gehäuse kann luftdicht abgeschlossen sein oder unter ständigem Überdruck stehen. Alternativ kann der Bereich der Kopplungseinrichtung, der teilweise innerhalb und teilweise außerhalb des Reinraums ist, beim Eindringen in den Reinraum immer wieder mit H2O2 beaufschlagt werden.

In einer bevorzugten Ausführungsform ist die Lagerungseinrichtung in einem horizontalen Abstand von höchstens 10 cm, bevorzugt 5 cm, besonders bevorzugt 3 cm, von der Blasform entfernt.

Vorteilhaft befindet sich die Lagerungseinrichtung in Richtung der Achse X zumindest teilweise auf Höhe der Blasform. Die Achse wird dabei insbesondere von einer Welle definiert, welche zumindest ein Seitenteil betätigt.

Es ist besonders vorteilhaft, die Lagerungseinrichtung so nahe wie möglich an die Stelle der Kopplungseinrichtung heranzuführen, an der durch die Verschwenkung der Seitenteile eine Kraft auf die Kopplungseinrichtung einwirkt. Die beschriebene Stelle an der Kopplungseinrichtung ist in der Regel in dem Bereich, in dem die Seitenteile angeordnet sind.

Bevorzugt ist eine Antriebseinrichtung vorgesehen, um das Bodenteil zu bewegen, sowie eine weitere Antriebseinrichtung, um die Seitenteile zu bewegen. Es wäre jedoch auch möglich, dass lediglich eine Antriebseinrichtung vorgesehen ist und über eine Kopplungseinrichtung eine Bewegung der Seitenteile an eine Bewegung des Bodenteils gekoppelt wird. Dabei ist es möglich, dass diese Kopplungseinrichtung wenigstens teilweise innerhalb des Reinraum angeordnet ist, es wäre jedoch auch möglich, dass die Kopplungseinrichtung vollständig außerhalb oder vollständig innerhalb des Reinraums vorgesehen ist.

Vorteilhaft ist wenigstens eine der außerhalb des Reinraums angeordneten Antriebseinrichtungen über eine Kopplungseinrichtung mit wenigstens einem Seitenteil oder mit dem Bodenteil gekoppelt und diese Kopplungseinrichtung erstreckt sich durch eine Grenze des Reinraums hindurch. Vorteilhaft wird der Reinraum durch eine Wandung begrenzt, wobei es sich hierbei um eine flexible oder eine starre Wandung handeln kann. Diese Wandung stellt dabei vorteilhaft auch die Grenze des Reinraums zu dem nicht sterilen Außenbereich dar. Unter einer Kopplungseinrichtung wird hier insbesondere eine mechanisch wirkende Einrichtung verstanden, welche eine Bewegung der Antriebseinrichtung auf das Bodenteil oder wenigstens ein Seitenteil überträgt.

Vorteilhaft weist die Dichtungseinrichtung ein elastisches Dichtmittel auf. So wäre es möglich, dass es sich bei der Kopplungseinrichtung beispielsweise um eine Stange oder ein Gestänge handelt und an diesem Gestänge ein Dichtmittel wie beispielsweise ein Faltenbalg angeordnet ist. Für die Verwendung eines derartigen Faltenbalgs kann trotz einer Bewegung der Kopplungseinrichtung eine dichte Grenze des Reinraums aufrecht erhalten werden. Vorteilhaft folgt wenigstens ein Abschnitt des Dichtmittels hinsichtlich seiner Bewegung der Kopplungseinrichtung.

Vorteilhaft bewegt die Antriebseinrichtung das Bodenteil oder wenigstens ein Seitenteil entlang eines endlichen Pfades, d. h. entlang eines Bewegungspfades, der wenigstens einen Endpunkt und vorteilhaft zwei Endpunkte aufweist (im Gegensatz etwa zu einer Bewegung entlang einer kreisförmigen oder elliptischen Bahn).

Vorteilhaft ist eine erste Antriebseinrichtung zum Bewegen des Bodenteils und eine zweite Antriebseinrichtung zum Bewegen des Seitenteils vorgesehen und beide Antriebseinrichtungen sind außerhalb des Reinraums angeordnet.

Bei einer weiteren vorteilhaften Ausführungsform weist die Dichteinrichtung eine Gassperre auf. In diesem Falle kann in einem abzudichtenden Bereich ein Gas, wie beispielsweise Wasserdampf eingeführt werden und dieser Wasserdampf eine Gasbarriere ausbilden.

Weiterhin wäre es jedoch auch möglich, dass eine Abdichtung durch Mittel wie beispielsweise sogenannte Wasserschlösser erfolgt. Dabei kann in einem Wasserbehälter ein bewegliches Element geführt werden, so dass eine Hubbewegung möglich ist, ohne dass hierbei eine Grenze zwischen einem sterilen und einem nicht sterilen Raum durch ein Gas überschritten werden kann.

Bei einer weiteren vorteilhaften Ausführungsform weist die Vorrichtung Zuführeinrichtungen zum Zuführen einer Temperierflüssigkeit für die Blasformen auf und Anschlüsse für die Zuführeinrichtungen sind innerhalb des Sterilraums bzw. Reinraums angeordnet. Weiterhin wäre es jedoch auch möglich, dass eine Reinraumgrenze so vorgesehen ist, dass die Zuführeinrichtungen zum Zuführen der Temperierflüssigkeit und die Anschlüsse für die Zuführeinrichtungen alle im Bodenteil stets außerhalb des Reinraums angeordnet sind. Vorteilhaft ist das Bodenteil und besonders bevorzugt sind auch die Seitenteile kühlwasserbeständig ausgeführt.

Vorteilhaft weist die Vorrichtung eine Sterilisationseinrichtung auf, welche vor den eigentlichen Blasstationen angeordnet ist und welche die einzelnen Kunststoffvorformlinge sterilisiert.

Weiterhin wird vorgeschlagen, ein Blasrad der Blasmaschine d. h. den Bereich, in dem aus dem Kunststoffvorformling ein Kunststoffbehältnis entsteht zu überarbeiten. Dabei wird ein Bereich um den eigentlichen formgebenden Bereich der Maschine, d. h. den Formträger mit der Blasform herum in einer Art gekapselt, dass dieser Bereich sterilisiert werden kann. Dabei wird vorteilhaft ein Sterilraum definiert und alle Medien und Bauteile, die die Grenzen dieses Sterilraums überqueren, werden so gestaltet, dass ein einmal bei Produktionsbeginn sterilisierter Raum auch steril bleibt. Durch die erfindungsgemäße Vorgehensweise ist es auch möglich, zu verhindern, dass Fett und Umgebungsluft in den Sterilraum eindringt.

In dem Bereich des Bodenteils ist es dabei erforderlich, eine Hubbewegung auszuführen, um ein geblasenes Behältnis formen zu können. Diese Hubbewegung kann dabei durch eine Kurve angesteuert werden, welche auch im Stand der Technik gefettet werden muss. Daher ist es vorteilhaft, die besagte Kurve außerhalb des Reinraums anzuordnen. Genauer gesagt ist es möglich, dass die besagte Kurve unterhalb des Formträgers und daher auch unterhalb des Reinraums verläuft.

Alternativ ist es auch möglich, das Behältnis auf dem Kopf stehend zu blasen, indem sich die besagte Kurve für das Bodenteil oberhalb des Formträgers und daher auch oberhalb des Reinraums befindet.

Es wären jedoch auch andere Antriebseinrichtungen, wie beispielsweise Servomotoren, hydraulische oder pneumatische Hubzylinder oder dergleichen als Antriebseinrichtung denkbar. Auch könnten Linearmotoren zum Einsatz kommen.

Die oben erwähnte Sterilraumgrenze zwischen dem Reinraum und der Umgebung wird vorteilhaft so gelegt, dass eine Antriebseinrichtung, beispielsweise die Kurve und Kurvenrolle, außerhalb des Sterilraums bleiben. Vorteilhaft wird der Reinraum nach unten hin durch eine durchbrochene und vorteilhaft solide Abgrenzung begrenzt.

Dieser Durchbruch dient, wie oben erwähnt, wenigstens zur Durchführung der Bodeneinheit der Blasstation, und ggfs. auch zur Durchführung der oben erwähnten Zu- und Abführungen der Temperiermedien für die Bodenform bzw. das Bodenteil.

Dabei kann zwischen dem Bodenteil und dem Durchbruch ein Falkenbalg vorgesehen sein, der einen Abschluss des Reinraums zum Bereich der Führungskurve hin gewährleistet.

Weiterhin wäre es möglich, an dem Durchbruch den besagten Falkenbalg durch eine geeignete Klemmvorrichtung z. B. in Art einer Schlauchschelle zu fixieren. Im Fall eines Produkt-oder Formenwechsels wäre es möglich, diese Seite lösbar auszuführen.

Dabei wäre es möglich, dass der besagte Faltenbalg in das Innere des Reinraums ragt, es wäre jedoch auch möglich, dass sich der Falkenbalg bezüglich des Reinraums nach Außen erstreckt.

Bei einer weiteren vorteilhaften Ausführungsform weist die Vorrichtung eine Beaufschlagungseinrichtung auf, welche die Kunststoffvorformlinge mit einem gasförmigen Medium beaufschlagt und diese Beaufschlagungseinrichtung ist innerhalb des Sterilraums angeordnet und weiterhin ist eine Antriebseinrichtung zum Bewegen der Beaufschlagungseinrichtung vorgesehen.

Bei dieser Beaufschlagungseinrichtung kann es sich beispielsweise um eine Blasdüse handeln, welche wie im Stand der Technik bekannt, zur Durchführung des Blasvorgangs an einer Mündung des Behältnisses oder auch an einer Blasform angelegt wird, um den Kunststoffvorformling insbesondere mit Druckluft zu beaufschlagen. Vorteilhaft ist die Antriebseinrichtung zum Bewegen der Beaufschlagungseinrichtung außerhalb des Sterilraums vorgesehen. Durch diese Vorgehensweise ist eine weitere Reduktion des Reinraumvolumens denkbar. Vorteilhaft bewegt sich eine Wandung, welche eine Grenze des Reinraums bildet mit den einzelnen Blasstationen mit.

Vorzugsweise sind in einer zu der Transportrichtung (der Behältnisse) senkrecht stehenden Richtung bestimmte Teile der Transporteinrichtung innerhalb des Reinraums angeordnet und andere Teile außerhalb.

Vorzugsweise handelt es sich bei der Transporteinrichtung um ein Transportrad, welches sich um eine vorgegebene Achse dreht, wobei wenigstens die Achse bzw. eine Welle der Transporteinrichtung außerhalb des Reinraums angeordnet ist. Damit wird erreicht, dass der Reinraum möglichst klein gehalten wird und damit auch das Innenvolumen des Reinraums gering gehalten werden kann. Weiterhin wird auf diese Weise auch erreicht, dass eine möglichst große Anzahl an Maschinenteilen, welche nicht unmittelbar mit den Blasstationen in Kontakt sind, außerhalb des Reinraums geführt werden können und auch auf diese Weise werden Kontaminationen gering gehalten.

Vorzugsweise weist der Reinraum wenigstens abschnittsweise ein ringförmiges Profil auf bzw. ein torusartiges Profil, wobei hier jedoch der Querschnitt dieses torusartigen Profils bevorzugt von einer Kreisform abweicht. Dies bedeutet, dass die Blasstationen durch die Transporteinrichtung auf einer im Wesentlichen kreisförmigen Bahn geführt werden.

Bei einer weiteren vorteilhaften Ausführungsform ist an jeder Blasstation eine Reckstange zum Recken der Kunststoffvorformlinge angeordnet. Diese Reckstange kann komplett im Reinraum angeordnet sein, bevorzugt innerhalb eines weiteren, an die Reckstange angepassten Gehäuses, das aus dem ringförmigen Reinraum herausragt. Alternativ kann die Reckstange wenigstens zeitweise und abschnittsweise aus dem Reinraum herausragen. Die Reckstange wird bekanntlich verwendet, um die Kunststoffvorformlinge im Rahmen des Herstellungsprozesses zu dehnen.

Vorzugsweise ist der Reinraum von mehreren Wandungen begrenzt und wenigstens eine dieser Wandungen ist gegenüber einer weiteren Wandung bewegbar und insbesondere drehbar angeordnet.

Vorzugsweise ist eine radial außen angeordnete Wandung des Reinraum stationär angeordnet. Damit kann eine Wandung mit einem insbesondere zylinderförmigen Außenprofil vorgesehen sein, welche den Reinraum begrenzt. Vom Inneren dieser Wandung ist eine weitere Wandung vorgesehen, die den Reinraum zu der anderen Seite hin begrenzt und die drehbar angeordnet ist. Vorzugsweise wird diese innen angeordnete Wandung mit den einzelnen Blasstationen gedreht. Vorzugsweise liegen sich die erwähnte drehbare Wand und die stationär angeordnete Wand gegenüber. Weiterhin wird der Reinraum von einer Wandung in Form eines Deckels begrenzt, wobei dieser Deckel vorzugsweise einteilig mit der drehbaren Wand ausgebildet ist.

Vorzugsweise ist zwischen wenigstens zwei Wandungen bzw. einer Wandung und einem Deckel eine Dichtungseinrichtung angeordnet. Diese Dichtungseinrichtung dichtet bevorzugt gegenüber einander bewegliche Teile gegeneinander ab. So wäre es beispielsweise möglich, zwischen einer Wandung und einem Deckel ein so genanntes Wasserschloss vorzusehen, bei dem ein hier bevorzugt ringförmiger Wasserkanal vorgesehen ist, in dem ein Abschnitt des gegenüber diesem Wasserkanal beweglichen Teils geführt wird.

Bei einer weiteren vorteilhaften Ausführungsform weist die Vorrichtung eine Zuführeinrichtung auf, um der Transporteinrichtung die Kunststoffvorformlinge zu übergeben und diese Zuführeinrichtung ist innerhalb des Reinraums angeordnet. Damit weist hier bevorzugt der Reinraum eine Ausbuchtung bzw. eine Abweichung von dem ansonsten kreisförmigen Querschnitt auf, und in dieser Ausbuchtung ist entsprechend die Zuführeinrichtung wie beispielsweise ein Transportstern für die Vorformlinge angeordnet. Auf diese Weise kann eine lückenlose Übergabe der Behältnisse von der Zuführeinrichtung an die Transporteinrichtung innerhalb eines Sterilraums erfolgen.

Bei einer weiteren vorteilhaften Ausführungsform weist die Vorrichtung auch eine Abführeinrichtung auf, um von der Transporteinrichtung die ausgebildeten Kunststoffbehältnisse zu übernehmen und diese Abführeinrichtung ist ebenfalls innerhalb des Reinraums angeordnet. Auf diese Weise kann auch beim Abtransport der Behältnisse ein Reinraum gewahrt werden. Damit ist es möglich, dass ein Einbringen von Vorformlingen aus vorgelagerten Aggregaten in den Isolator bzw. Reinraum sowie ein Ausbringen der Flaschen zu einem nächsten Aggregat unter Reinraumbedingungen möglich ist. Der Isolator bzw. Reinraum kann mit Reinigungs- und Sterilisationsmedien beaufschlagt werden.

Bei einer weiteren vorteilhaften Ausführungsform ist innerhalb des Reinraums ein Sterilgas vorgesehen und dieses Sterilgas steht unter einem Druck, der höher ist als ein Druck außerhalb des Reinraums. Damit kann der Reinraum optional durch das Einbringen von sterilisierter Luft auf einem höheren Druckniveau als die Umgebung gehalten werden, wodurch das Eindringen von Mikroorganismen verhindert werden kann. Weiterhin besteht die Möglichkeit, dem Reinraum kontinuierlich einen antimikrobiellen Wirkstoff zuzuführen und auf diese Weise ein hygienisches Umfeld aufrecht zu erhalten.

Durch einen räumlich begrenzten Reinraum in der Blasvorrichtung ist es möglich, vorher entkeimte Vorformlinge ohne Rekontamination sowohl außen als auch innen während des Streckblasvorgangs zum Füller zu transportieren. Des Weiteren ist der Reinraum im Vergleich zur gesamten Umformungsvorrichtung einfacher auf einem keimarmen Level zu halten.

Die vorliegende Erfindung ist weiterhin auf eine Anlage zum Herstellen von Kunststoffbehältnissen gerichtet, welche eine Vorrichtung zum Umformen von Kunststoffbehältnissen der oben beschriebenen Art aufweist, sowie eine Heizeinrichtung, wobei diese Heizeinrichtung an einer Transportrichtung der Kunststoffvorformlinge stromaufwärts bezüglich der Vorrichtung angeordnet ist.

Diese Heizeinrichtung dient dazu, um die Vorformlinge zu erwärmen, damit diese anschließend in einem Blasvorgang zu Behältnissen expandiert werden können. Stromabwärts bzw. nach der Umformungsvorrichtung ist eine Fülleinrichtung vorgesehen, welche die Behältnisse mit einem Getränk, insbesondere mit einem sterilisierten Produkt, befüllt. Auch diese Fülleinrichtung ist dabei in einem Reinraum angeordnet. Weiterhin erstreckt sich bevorzugt der Reinraum bis in den Bereich einer Verschließeinrichtung, welche die Behältnisse mit einem Verschluss verschließt.

Weiterhin weist die Anlage bevorzugt eine Sterilisationseinrichtung auf, welche wenigstens einen Bereich der Kunststoffvorformlinge vor Erreichen der Vorrichtung sterilisiert.

Dabei kann diese Sterilisation mit einem gasförmigen Medium, wie insbesondere gasförmigen Wasserstoffperoxid, durchgeführt werden. Es wäre jedoch auch möglich, dass die Sterilisation unter Verwendung von Strahlung, wie beispielsweise Elektronenstrahlen und/oder UV-Licht bewirkt wird. Dabei ist bevorzugt eine Sterilisationseinrichtung vorgesehen, welche insbesondere auch die Innenoberfläche der Kunststoffvorformlinge sterilisiert. Daneben kann jedoch auch die Außenoberfläche der Kunststoffvorformlinge sterilisiert werden.

Vorteilhaft weist die Anlage einen weiteren Reinraum auf, der in der Transportrichtung der Kunststoffvorformlinge vor der Vorrichtung angeordnet ist.

Vorzugsweise geht dieser weitere Reinraum in den Reinraum der Umformungseinrichtung über. Auf diese Weise ist es möglich, die Kunststoffbehältnisse ausgehend von deren Sterilisation kontinuierlich bis zum Verschließen zu fördern und gleichwohl die hierzu nötigen Reinräume relativ gering zu halten. Damit ist bevorzugt der Reinraum als sich von der Sterilisationseinrichtung bis zu der Verschließeinrichtung erstreckender Kanal vorgesehen, der besonders bevorzugt jeweils an die entsprechenden Blasstationen oder Halteeinrichtungen wie Greifelemente für die Vorformlinge oder Kunststoffbehältnisse angepasst ist. Der Kanal kann auch durch größere Räume unterbrochen werden, beispielsweise zur Anordnung von Transportsternen, welche bis auf ihren Rotationsantrieb im Wesentlichen vollständig im Reinraum angeordnet werden können.

Dabei weisen die Blasstationen jeweils Blasformen auf, wobei die Blasformen zwei Seitenteile und ein Bodenteil aufweisen. Die Seitenteile und das Bodenteil bilden gemeinsam einen Hohlraum aus, innerhalb dessen der Kunststoffvorformling zum Kunststoffbehältnis umgeformt wird. Die den Hohlraum begrenzenden Bereiche der Seitenteile und des Bodenteils und bevorzugt die gesamten Seitenteile und das Bodenteil sind dabei stets innerhalb des Reinraums angeordnet. Vorteilhaft werden wenigstens die Seitenteile oder das Bodenteil in einer Antriebseinrichtung bewegt, die sich wenigstens teilweise und bevorzugt vollständig außerhalb des Sterilraums befindet.
Unter dem Begriff Blasform werden nicht nur die beiden Blasformhälften verstanden, sondern der gesamte Formträger inklusive der jeweiligen Schalen, wie z.B. Mutterform oder Formträgerschale. Üblicherweise umfasst ein Seitenteil mindestens einen Formträger und eine Blasformhälfte.

Bevorzugt wird auch wenigstens ein Bereich der Transporteinrichtung außerhalb des Reinraums bewegt. Bei einem weiteren bevorzugten Verfahren werden die Blasstationen auf einer kreisförmigen Bahn bewegt und besonders bevorzugt ständig innerhalb des Reinraums bewegt.

Weitere Vorteile und Ausführungsformen ergeben sich aus den beigefügten Zeichnungen:

Darin zeigen:
- Fig. 1: eine schematische Darstellung einer Anlage zum Herstellen von Kunststoffbehältnissen nach dem Stand der Technik;
- Fig. 2: eine Draufsicht eines Reinraums im Bereich einer Blasstation, nach dem Stand der Technik;
- Fig. 3: eine Seitenansicht eines Reinraums im Bereich einer Blasstation, nach dem Stand der Technik;
- Fig. 4: eine Seitenansicht einer erfindungsgemäßen Vorrichtung;
- Fig. 5: eine Seitenansicht einer erfindungsgemäßen Vorrichtung in einer weiteren Ausführungsform; und
- Fig. 6: eine Seitenansicht einer erfindungsgemäßen Vorrichtung in einer weiteren Ausführungsform.

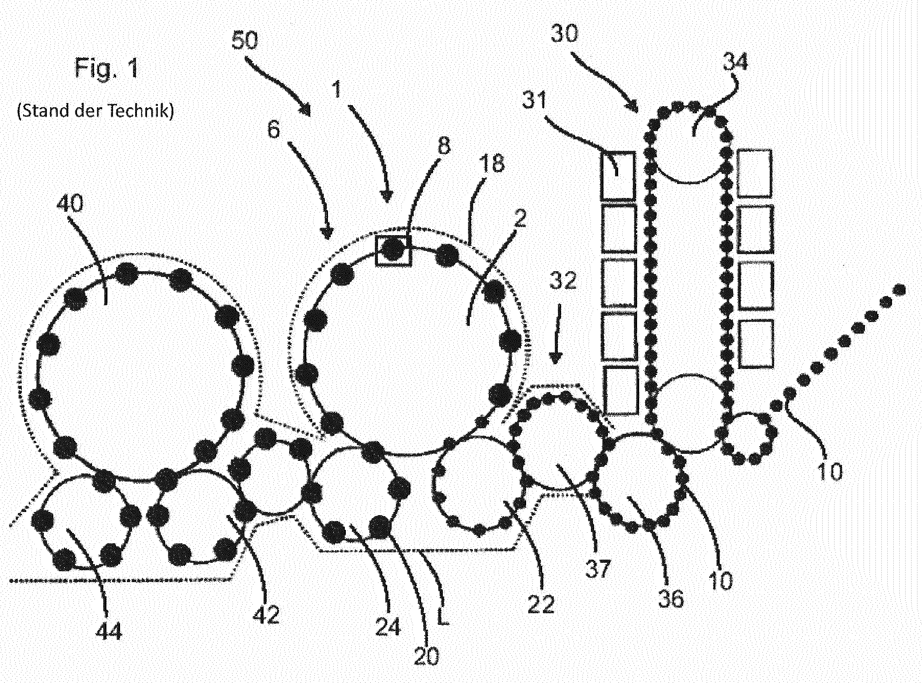

Figur 1 zeigt eine schematische Darstellung einer Anlage nach dem Stand der Technik zum Herstellen von Kunststoffbehältnissen. Diese Anlage 50 weist eine Heizeinrichtung 30 auf, in der Kunststoffvorformlinge 10 erwärmt werden. Dabei werden diese Kunststoffvorformlinge 10 mittels einer Transporteinrichtung 34, wie hier einer umlaufenden Kette, durch diese Heizeinrichtung 30 geführt und dabei mit einer Vielzahl von Heizelementen 31 erwärmt. An diese Heizeinrichtung 30 schließt sich eine Übergabeeinheit 36 an, welche die Vorformlinge 10 an eine Sterilisationseinrichtung 32 übergibt. Diese Sterilisationseinrichtung 32 weist dabei hier ebenfalls ein Transportrad 37 auf und an diesem Transportrad 37 oder auch stationär können Sterilisationselemente angeordnet sein. In diesem Bereich ist beispielsweise eine Sterilisation durch Wasserstoffperoxidgas oder auch, wie oben erwähnt, durch elektromagnetische Strahlung möglich. Insbesondere wird in diesem Bereich eine Innensterilisation der Vorformlinge durchgeführt. Bei elektromagnetischer Strahlung ist es vorteilhaft, dass zur Innensterilisation Strahlungsfinger in die Vorformlinge 10 eintauchen. Weiterhin können ein oder mehrere zusätzliche Strahler vorgesehen sein, die die Vorformlinge 10 von außen sterilisieren. Bei der Verwendung von Wasserstoffperoxid können eintauchende Düsen zur Innensterilisation vorgesehen sein oder alternativ Düsen, die insbesondere zwar nicht eintauchen, sich jedoch mit den Vorformlingen 10 mitbewegen. Zur Außensterilisation können weitere Düsen unterhalb der Vorformling 10 vorgesehen sein.

Das Bezugszeichen 6 bezeichnet in seiner Gesamtheit einen Reinraum, dessen Außenbegrenzungen hier durch die punktierte Linie L angedeutet ist. Man erkennt, dass dieser Reinraum 6 in dem Bereich der Sterilisationseinheit 32 beginnt. In diesem Bereich können Schleuseneinrichtungen vorgesehen sein, um die Kunststoffvorformlinge in den Reinraum 6 einzuführen, ohne dass dabei zu viel Gas innerhalb des Reinraums verloren geht.
Es ist jedoch auch denkbar, die Sterilisationseinheit 32 bereits vor und innerhalb der Heizeinrichtung 30 vorzusehen und demnach die Heizeinrichtung 30 auch innerhalb des Reinraums anzuordnen.

Der Reinraum ist, wie durch die gestrichelte Linie L angedeutet, an die Außengestalt der einzelnen Anlagenkomponenten angepasst. Auf diese Weise kann das Volumen des Reinraums reduziert werden.

Das Bezugszeichen 1 bezeichnet in ihrer Gesamtheit eine Umformungsvorrichtung, bei der an einem Transportrad 2 eine Vielzahl von Blasstationen 8 angeordnet ist, wobei hier lediglich einer dieser Blasstationen 8 dargestellt ist. Mit diesen Blasstationen 8 werden die Kunststoffvorformlinge 10 zu Behältnissen 20 expandiert. Obwohl hier nicht detailliert gezeigt, befindet sich nicht der gesamte Bereich des Transporteinrichtung 2 innerhalb des Reinraums 6, sondern der Reinraum 6 bzw. Isolator ist gewissermaßen als Mini-Isolator innerhalb der gesamten Vorrichtung realisiert. So wäre es möglich, dass der Reinraum zumindest im Bereich der Umformungsvorrichtung 1 kanalartig ausgeführt ist.

Das Bezugszeichen 22 bezieht sich auf eine Zuführeinrichtung, welche die Vorformlinge an die Umformungseinrichtung 1 übergibt und das Bezugszeichen 24 auf eine Abführeinrichtung, welche die hergestellten Kunststoffbehältnisse 20 von der Umformungsvorrichtung 1 abführt. Man erkennt, dass der Reinraum 6 in dem Bereich der Zuführeinrichtung 22 und der Abführeinrichtung 24 an jeweils Ausnehmungen aufweist, welche diese Einrichtungen 22, 24 aufnehmen. Auf diese Weise kann in besonders vorteilhafter Weise eine Übergabe der Kunststoffvorformlinge 10 an die Umformungsvorrichtung 1 bzw. eine Übernahme der Kunststoffbehältnisse 20 von der Umformungsvorrichtung 1 erreicht werden.

Mit einer Übergabeeinheit 42 werden die expandierten Kunststoffbehältnisse an eine Befüllungseinrichtung 40 übergeben und von dieser Befüllungseinrichtung 40 anschließend über eine weitere Transporteinheit 44 abgeführt. Dabei befindet sich auch die Befüllungseinrichtung 40 innerhalb des besagten Reinraums 6. Auch im Falle der Befüllungseinrichtung wäre es möglich, dass nicht die gesamte Befüllungseinrichtung 40 mit beispielsweise einem Reservoir für ein Getränk vollständig innerhalb des Reinraums 6 angeordnet sind, sondern auch hier lediglich diejenigen Bereiche, in denen tatsächlich die Behältnisse geführt werden. Insoweit könnte auch die Befüllungseinrichtung in ähnlicher Weise aufgebaut sein wie die Vorrichtung 1 zum Umformen von Kunststoffvorformlingen 10.

Wie erwähnt, ist der Reinraum 6 im Bereich der Vorrichtung 1 auf einen geringst möglichen Bereich reduziert, nämlich im Wesentlichen auf die Blasstationen 8 selbst. Durch diese kleinbauende Gestaltung des Reinraums 6 ist es leichter und schneller möglich, einen Reinraum überhaupt herzustellen und auch die Sterilhaltung in der Betriebsphase ist weniger aufwändig. Auch wird weniger Sterilluft benötigt, was zu kleineren Filteranlagen führt und auch die Gefahr von unkontrollierter Wirbelbildung wird reduziert.

Wie oben bereits erwähnt, ist die Heizeinrichtung zum Erwärmen der Kunststoffvorformlinge bevorzugt ebenfalls aseptisch ausgeführt. Dies bedeutet, dass die Kunststoffvorformlinge bereits im Bereich der Heizeinrichtung 30, anders als in Fig. 1 gezeigt, durch einen Reinraum geführt werden können und sich dieser Reinraum beispielsweise durchgehend über die Blasmaschine bis zu dem Füller erstreckt. Dabei ist es möglich, dass die vollständige Heizeinrichtung 30 innerhalb eines Sterilraums angeordnet ist, es wäre jedoch auch möglich, dass auch hier insbesondere der Bereich, in dem die Kunststoffvorformlinge transportiert werden, gegenüber der Umgebung als Sterilraum gekapselt ist. So wäre es beispielsweise möglich, dass die Kunststoffvorformlinge mittels Dornen transportiert werden, die in ihre Mündung eingreifen und die Dornen dabei durch eine Wandung in einen Reinraum hineinragen. Dieser Reinraum könnte ebenfalls mit einem Überdruck beaufschlagt werden, so dass keine Umgebungsluft in diesen Reinraum eindringen kann.

Die Heizeinrichtung könnte dabei als Infrarotheizeinrichtung ausgestaltet sein, wie in Fig. 1 dargestellt. Vorteilhaft wird jedoch hier als Heizeinrichtung eine Mikrowellenheizeinrichtung verwendet. Derartige Mikrowellenheizeinrichtungen zum Erwärmen von Kunststoffvorformlingen sind aus dem Stand der Technik an sich bekannt. Dabei könnte eine Vielzahl von Mikrowellenheizstationen beispielsweise an einem Trägerrad angeordnet sein. Über Schleusen könnten die Kunststoffvorformlinge diesen einzelnen Heizstationen zugeführt werden. Aufgrund der Ausführung mit einzelnen Heizstationen eignet sich eine mikrowellenbasierte Heizeinrichtung in besonderer Weise für die Kombination mit Sterilräumen.

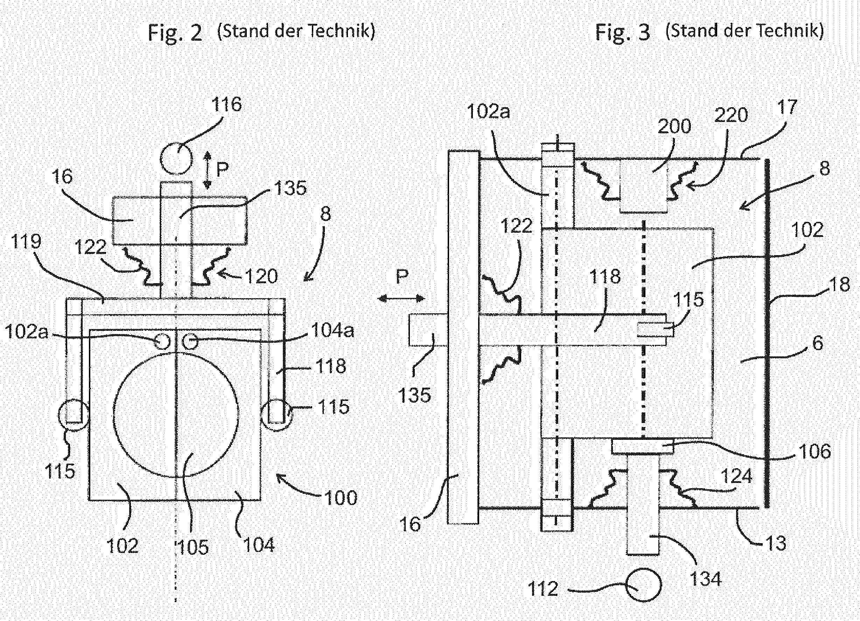

Fig. 2 zeigt eine Draufsicht einer Blasstation 8 aus dem Stand der Technik. Diese Blasstation weist dabei eine (stark vereinfacht und ohne Träger dargestellte) Blasform 100 mit einem ersten Seitenteil 102, sowie einem zweiten Seitenteil 104 auf, welche hier jeweils um Schwenkachsen 102a und 104a schwenken. Die Schwenkachsen 102a und 104a selbst sind feststehend relativ zu den (nicht gezeigten) Wandungen 13, 17 angebracht. In ihrem Inneren bilden die beiden Seitenteile einen Hohlraum 105 aus, in dem Kunststoffvorformlinge zu Kunststoffbehältnissen expandiert werden können. An den beiden Seitenteilen 102 und 104 sind dabei jeweils über Gelenke 115 Arme 118 angeordnet, wobei diese Arme wiederum an einem Hauptarm 119 vorgesehen sind. Das Bezugszeichen 116 bezieht sich auf eine Antriebseinrichtung, welche eine Stange 135 in der Richtung des Doppelpfeils P bewegt, welche damit auch die Arme 118 und 119 bewegt. Durch die Bewegung dieser Stange 135 können die beiden Seitenteile 102 und 104 um die Schwenkachsen 102a, 104a auseinander bzw. zusammengeschwenkt werden. Insbesondere dienen die Arme 118 und 119 zur Verriegelung der Seitenteile miteinander.

Das Bezugszeichen 120 kennzeichnet eine Dichtungseinrichtung mit einem Faltenbalg 122, der einerseits an der Kopplungseinrichtung 135 und andererseits an einer Begrenzungswand 16 angeordnet ist. Diese Begrenzungswand 16 trennt dabei den Reinraum, in dem die Blasstation 8 angeordnet ist von dem nichtsterilen Raum, in dem die Antriebseinrichtung 116 vorgesehen ist, ab. Damit ist hier eine Dichtungseinrichtung 120 vorgesehen, die einen Öffnungsmechanismus, der der Blasform bzw. dem Formenträger zugeordnet ist, von dem Transportweg der Behältnisse oder Vorformlinge abschirmt.

Der Faltenbalg 122 besteht aus einem Material, welches aus einer Gruppe von Materialien ausgewählt ist, welche Kunststoffe, EPDM, Kautschuk, Elastomere, Gummi oder Stahl enthält. Der Faltenbalg 122 kann dabei als Membranbalg oder Wellbalg oder als Kombination hieraus ausgebildet sein.

Fig. 3 zeigt eine Seitendarstellung der in Fig. 2 gezeigten Vorrichtung nach dem Stand der Technik. Bei der in Fig. 3 gezeigten Darstellung erkennt man zusätzlich eine Antriebseinrichtung 112, welche eine Kopplungseinrichtung 134 bewegt, wobei an der Kopplungseinrichtung 134 wiederum ein Bodenteil 106 angeordnet ist, welches ebenfalls zum Abschließen des Hohlraums 105 dient. Das Bezugszeichen 13 kennzeichnet eine untere Wandung des in seiner Gesamtheit mit 6 bezeichneten Reinraums, durch welche sich die Kopplungseinrichtung 134 hindurch erstreckt. Das Bezugszeichen 124 kennzeichnet wieder eine Dichtungseinrichtung wie einen Faltenbalg, der diese Bewegung der Kopplungseinrichtung 134 gegenüber der Wandung 13 abdichtet.

Das Bezugszeichen 18 kennzeichnet wieder eine radial außenliegende Wandung, welche hier stehend angeordnet ist. Die Wandungen 13 und 17, sowie auch die Wandung 16 drehen sich mit den einzelnen Blasstationen 8 mit. Man erkennt, dass der Reinraum 6 relativ eng um die Blasstationen herum gebaut ist, sodass ein sehr geringes Volumen steril gehalten werden muss. Insgesamt sind hier drei Dichtungseinrichtungen 122, 124, 220 vorgesehen, genauer auch eine weitere Dichtungseinrichtung 220, welche zum Abdichten der Bewegung einer Blasdüse 200 dient. Eine weitere Kopplungseinrichtung 135 koppelt die Seitenteile 102, 104 an die Antriebseinrichtung 116. Das Bezugszeichen 102a kennzeichnet wiederum die Schwenkachse zum Schwenken des Seitenteils 102, welche die Wandungen 13, 17 durchdringt und unbeweglich an diesen Wandungen 13, 17 befestigt ist.

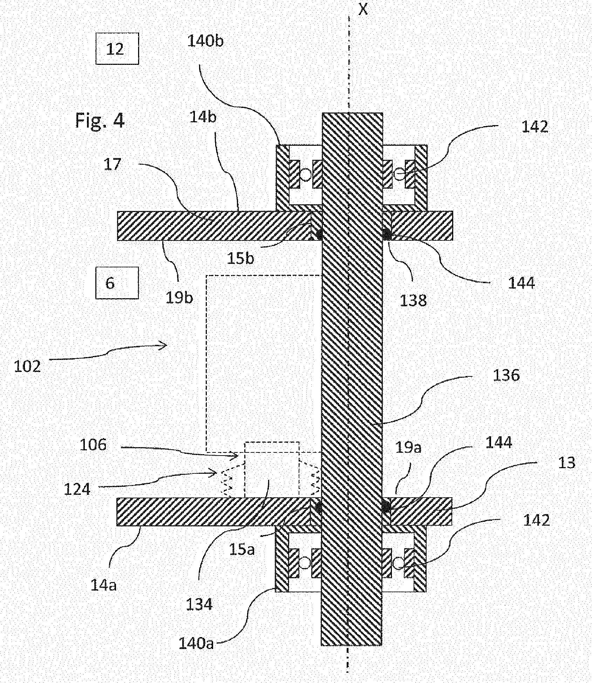

Fig. 4 zeigt eine Seitenansicht einer erfindungsgemäßen Vorrichtung. Rein schematisch sind ein Seitenteil 102 einer Blasform 100 sowie ein Bodenteil 106 gezeigt. Nicht gezeigt, aber vorhanden, ist noch ein weiteres Seitenteil 104, welches in der Bildebene hinter dem Seitenteil 102 liegt. Das Bodenteil 106 ist an einer Kopplungseinrichtung 134 angebracht, welche durch eine Ausnehmung in der Wandung 13 hindurch geführt wird, um eine Bewegung des Bodenteils 106 parallel zur Achse X, welche die Drehachse der Kopplungseinrichtung 136 darstellt, zu ermöglichen. Die Achse X ist weiterhin im Wesentlichen senkrecht zu der oberen und unteren Wandung 13, 17 angeordnet.
Die Bewegung des Bodenteils 106 ist notwendig, um die Blasform 100 komplett zu verschließen bzw. um die fertig geblasenen Flaschen aus der Blasform 100 entnehmen zu können. Die Bewegung der Kopplungseinrichtung 134 ohne Verschmutzung des Reinraums 6 wird hierbei durch einen Faltenbalg 124 ermöglicht, welcher den Reinraum 6 von der Umgebung 12 abgrenzt.

Weiterhin ist in Fig. 4 eine Kopplungseinrichtung 136, die sogenannte Hauptwelle, gezeigt. Die Hauptwelle 136 verläuft dabei durch die Wandungen 13 und 17 und ist jeweils im Bereich der Wandungen 13, 17, welche den Reinraum begrenzen, mit einer Dichtungseinrichtung 144 gegenüber der Lagerungseinrichtung 140 a,b abgedichtet.
Die Lagerungseinrichtung 140 a,b und auch die Kopplungseinrichtung 136 sind dabei im Wesentlichen senkrecht zu den Wandungen 13, 17 angeordnet.

Die Dichtungseinrichtung 144 ist bevorzugt als Grenze zum Reinraum 6 angebracht, so dass sich die Lagerungseinrichtungen 140 a,b außerhalb des Reinraums 6 befinden.
Lediglich die Fläche 138 der Lagerungseinrichtung 140 a,b dient zusammen mit der Wandung 13, 17 als Begrenzung des Reinraums. Diese Fläche 138, die das innerste Ende der Lagerungseinrichtung 140 a,b darstellt, liegt höchstens in der Ebene des Innenbereichs 19 a,b der Wandung 13, 17, erstreckt sich aber von dort aus nicht weiter in Richtung des Reinraums.
Es wäre alternativ auch denkbar, die Lagerungseinrichtung 140 a,b aseptisch auszuführen und dann innerhalb des Reinraums 6 anzuordnen.

Die Lagerungseinrichtung 140 a,b ist direkt mit der Wandung 13 bzw. 17 verbunden, bevorzugt verschweißt oder verschraubt. Die Lagerungseinrichtung 140 a,b ist somit unbeweglich an der Wandung 13, 17 befestigt und dient der Abstützung der drehbaren Hauptwelle 136. Vorteilhafterweise wird beispielsweise die Lagerungseinrichtung 140 b an der Wandung 17 verschraubt und die zweite Lagerungseinrichtung 140 a an der Wandung 13 verschweißt. Dadurch ist es möglich, die Hauptwelle 136 nicht überzubestimmen, sondern die Lagerungseinrichtung 140 b an der Wandung 17 nach Bedarf einzustellen.

Zwischen der Lagerungseinrichtung 140 a,b und der Hauptwelle 136 sind weiterhin ein oder mehrere Lagerungsmittel 142, wie beispielweise Kugellager vorgesehen, um eine Drehbewegung der Hauptwelle 136 um die Achse X zu ermöglichen. Diese Lager müssen nicht aseptisch ausgeführt sein, da sie sich außerhalb des Reinraums befinden.

Die Lagerungseinrichtung 140 b ist in Fig. 4 an dem Außenbereich 14b der Wandung 17 und zudem an dem Seitenbereich 15b, also parallel zur Achse X, an der Wandung 17 befestigt, bevorzugt verschraubt.
Alternativ ist es möglich, die Lagerungseinrichtung 140 b lediglich an dem Außenbereich 17b einer Wandung 17 anzubringen. Bevorzugt ist die Lagerungseinrichtung 140 b jedoch nur in dem Seitenbereich 15b an der Wandung 17 angebracht, der parallel zur Achse X ist.
Das gleiche gilt spiegelverkehrt für die Befestigung der Lagerungseinrichtung 140 a an der Wandung 13. Bevorzugt wird die Lagerungseinrichtung 140 a jedoch an der Wandung 13 verschweißt.

Die obere Wandung 17, bzw. Fläche 19b der Wandung 17 ist insbesondere parallel zu der unteren Wandung 13, bzw. deren Fläche 19a.

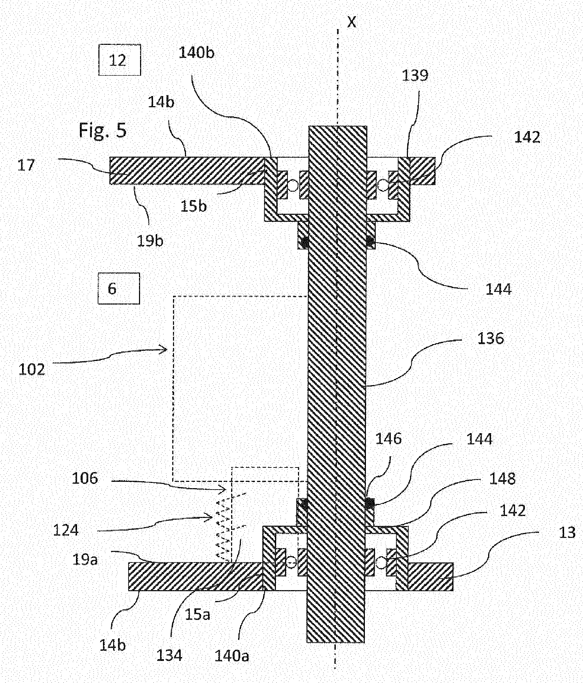

Fig. 5 zeigt eine weitere Ausführungsform einer erfindungsgemäßen Vorrichtung. Analog zur Fig. 4 sind rein schematisch ein Seitenteil 102 einer Blasform 100 sowie ein Bodenteil 106 gezeigt. Weiterhin sind die Wandungen 13 und 17 dargestellt, die den Reinraum 6 nach oben bzw. unten hin begrenzen. Die Hauptwelle 136 durchdringt die Wandung 17 nach oben sowie die Wandung 13 nach unten. Im Gegensatz zur Fig. 4 sind in der Fig. 5 Lagerungseinrichtungen 140 a,b gezeigt, die jeweils von den Wandungen 13 und 17 beginnend in den Reinraum 6 hinein ragen.

Die Lagerungseinrichtung 140 a ist dabei an dem Seitenbereich 15a, welcher parallel zur Achse X ist, der Wandung 13 befestigt, bevorzugt verschweißt. Bei dieser Parallelität ist insbesondere die Mittelachse des Lochs gemeint, welches den Seitenbereich 15 hervorruft. Es kann sich aber auch um die Innenfläche handeln. Zwischen der Lagerungseinrichtung 140 a und der Hauptwelle 136 sind ein oder mehrere Lagerungsmittel 142, wie z.B. Kugellager, sowie an der Seite der Lagerungseinrichtung 140 a, die an den Reinraum 6 anschließt, eine Dichtungseinrichtung 144 angebracht.
Die Dichtungseinrichtung 144 ermöglicht eine Rotationsbewegung der Hauptwelle 136 und dichtet dabei gleichzeitig den Reinraum 6 zur Umgebung 12 ab.
Analog zur Lagerungseinrichtung 140 a an der Wandung 13 ist eine weitere Lagerungseinrichtung 140 b an der Wandung 17 angebracht, bevorzugt angeschraubt.
Diese Lagerungseinrichtung 140 b ist in Fig. 4 ebenso am Seitenbereich 15b der Wandung 17 befestigt.
Alternativ können eine oder beide Lagerungseinrichtungen 140 a,b lediglich am Innenbereich 19 a,b der Wandungen 13, 17 angebracht sein. Bevorzugt ist zumindest eine der beiden Lagerungseinrichtungen 140 a,b sowohl am Innenbereich 19 a oder 19b als auch am Seitenbereich 15a oder 15b befestigt.
Das äußerste Ende der Lagerungseinrichtung 140 a,b stellt die Fläche 139 dar. Die äußerst mögliche Position dieser Fläche 139 ist in der Ebene des Außenbereichs 14 b. Die Fläche 139 kann sich aber auch weiter innen befinden. Die Position dieser Fläche, die am weitesten innen ist, ist in der Ebene des Innenbereichs 19b.
Die Fläche 139 ist also in Richtung der Achse X im Bereich zwischen der Ebene des Außenbereichs 14, a,b und der Ebene des Innenbereichs 19 a,b angeordnet.
Im laufenden Betrieb der Vorrichtung 1 sind die Lagereinrichtungen 140 a,b immer unbeweglich mit den Wandungen 13 bzw. 17 verbunden.

Die Wandungen 17, 13 sind insbesondere jeweils in einer horizontalen Ebene angeordnet.

Es ist aber genauso denkbar, die Lagerungseinrichtung 140 b an der Wandung 17 zu verschweißen und die Lagerungseinrichtung 140 a an der Wandung 13 zu verschrauben bzw. beide zu verschweißen oder beide zu verschrauben. Gleiches gilt für die in Fig. 4 gezeigte Ausführungsform.

Die Lagerungseinrichtung 140 a weist weiterhin in Fig. 4 zwei Flächen 146, 148 auf. Diese Flächen 146, 148 sind bevorzugt in einem Winkel zur Horizontalen (nicht gezeigt) ausgeführt, besonders bevorzugt in einem Winkel von 5°-45°zur Horizontalen, damit Flüssigkeiten, wie beispielsweise Kondensat oder flüssige oder schaumförmige Reinigungs- bzw. Sterilisationsmittel abfließen können.
Die Lagerungseinrichtung 140 a weist bevorzugt nur eine horizontale Fläche 146 auf, da die Lagerungseinrichtung 140 a besonders bevorzugt rohrförmig ausgebildet ist.

Alternativ kann die Lagerungseinrichtung 140a (bzw. auch die Lagerungseinrichtung 140b) auch in Form eines hexagonalen Hohlzylinders ausgebildet sein bzw. sie kann eine hexagonale Außenform und eine runde Innenform aufweisen.

Insbesondere kann zwischen einem, bevorzugt jedem, Lagerungsmittel 142 und der Dichtungseinrichtung 144 eine weitere Dichtung (nicht gezeigt) vorhanden sein, welche lediglich das im Lagerungsmittel eingesetzte Fett oder Öl im Lagerungsmittel hält.

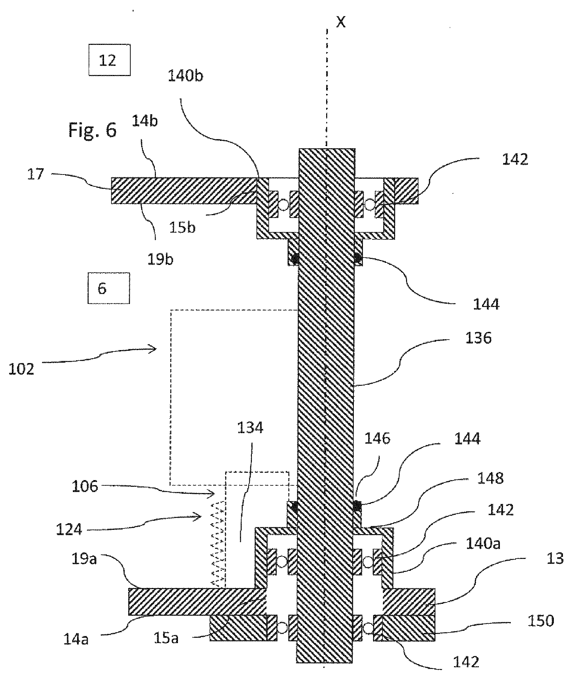

Fig. 6 zeigt eine dritte, erfindungsgemäße Ausführungsform der Vorrichtung 1. Die Lagerungseinrichtung 140a ist vom Prinzip her genauso wie in Fig. 5 beschrieben, dargestellt. Sie ist jedoch nur am Innenbereich 19a der Wandung 13 befestigt, bevorzugt verschraubt oder verschweißt. Alternativ kann sie natürlich analog zur Fig. 4 angebracht werden.
Zusätzlich zur Lagerungseinrichtung 140a dient noch eine weitere Abstützungseinrichtung 150 zur Lagerung bzw. Führung der Hauptwelle 136. Im Bereich der Abstützungseinrichtung sind auch ein oder mehrere Lagerungsmittel 142, beispielsweise Kugellager vorgesehen. Die Abstützungseinrichtung 150 ist am Außenbereich 14a der Wandung 13 befestigt. Die Abstützungseinrichtung 150 kann ebenso rohrförmig ausgebildet sein, bevorzugt weist sie aber eine davon abweisende, beispielsweise eckige Gestalt auf. Besonders bevorzugt ist die Abstützungseinrichtung 150 an die (nicht gezeigte) Ausnehmung in der Wandung 13 angepasst, die zum Austauschen bzw. Einsetzen der gesamten Blasstation 8 aus dem bzw. in den Reinraum 6 dient. Die Abstützeinrichtung 150 stellt ein separates Bauteil dar.

Es wird darauf hingewiesen, dass auch Kombinationen der in den Fig. 4-6 gezeigten Ausführungsformen mit unter Schutz gestellt sind. Beispielsweise kann eine Lagerungseinrichtung 140 a,b innerhalb des Reinraums 6 angebracht sein, wohingegen sich die andere Lagerungseinrichtung 140a,b außerhalb des Reinraums befindet. Auch ist es denkbar, auf einer Seite die Lagerungseinrichtung 140a,b mit der zusätzliche Abstützungseinrichtung 150 zu versehen und auf der anderen Seite lediglich eine Lagerungseinrichtung 140 a,b außer- oder innerhalb des Reinraums 6 vorzusehen.
Weiterhin ist selbstverständlich auch eine derartige Ausführungsform umfasst, bei der die Kopplungseinrichtung nicht auf beiden Seiten durch den Reinraum 6 geht, sondern lediglich auf einer Seite, beispielsweise nur durch die Wandung 13 oder nur durch die Wandung 17. Dabei ist dann nur eine Lagerungseinrichtung 140 a,b vorgesehen.
Lediglich durch die Wandung 13 treten z.B. die Kopplungseinrichtung 134 für das Bodenteil 106 oder die (nicht gezeigte) Verriegelungswelle, wohingegen von oben, also durch die Wandung 17, unter anderem die Blasdüse 200 tritt.
Als Dichtungseinrichtungen wurden im Rahmen der Figuren überwiegend Rotationsdichtungen dargestellt. Es kann sich jedoch auch um Faltenbalge eine Gassperre, Dampfsperre, eine Dampfkondensator- oder eine Flüssigkeitssperre, beispielsweise ein Wasserschloß handeln. Die Dichtungseinrichtung ist weiterhin vorteilhaft temperaturbeständig.

### Bezugszeichenliste

- 1: Umformungsvorrichtung
- 2: Transportrad
- 6: Reinraum
- 8: Blasstation
- 10: Kunststoffvorformlinge
- 12: Umgebung
- 13: Boden, untere Wandung
- 14 a,b: Außenbereich der Wandung
- 15 a,b: Seitenbereich der Wandung
- 16: Seitenwand, Begrenzungswand
- 17: Deckel, Wandung
- 18: Wandung
- 19 a,b: Innenbereich der Wandung
- 20: Kunststoffbehältnis
- 22: Zuführeinrichtung
- 24: Abführeinrichtung
- 30: Heizeinrichtung
- 31: Heizelemente
- 32: Sterilisationseinrichtung
- 34: Transporteinrichtung
- 36: Übergabeeinheit
- 40: Befüllungseinrichtung
- 42: Übergabeeinheit
- 44: Transporteinheit
- 50: Anlage
- 100: Blasform
- 102: Seitenteil
- 102a,104a: Schwenkachsen
- 104: Seitenteil
- 105: Hohlraum
- 106: Bodenteil
- 112: Antriebseinrichtung (Boden)
- 115: Gelenke
- 116: Antriebseinrichtung (Formträger)
- 118: Arme
- 119: Hauptarm
- 120, 220: Dichtungseinrichtung
- 122, 124: Faltenbalg
- 134, 135: Kopplungseinrichtung, Kraftübertragungseinrichtung
- 136: Kopplungseinrichtung, Hauptwelle
- 138, 139: Fläche
- 140 a,b: Lagerungseinrichtung
- 142: Lagerungsmittel
- 144: Dichtungseinrichtung
- 146, 148: Fläche
- 150: Abstützungseinrichtung
- 200: Beaufschlagungseinrichtung, Blasdüse

- X: Achse

## Patentansprüche

1. Vorrichtung (1) zum Umformen von Kunststoffvorformlingen (10) zu Kunststoffbehältnissen (20) mit einer Transporteinrichtung (2), an der eine Vielzahl von Blasstationen (8) angeordnet ist, wobei jede dieser Blasstationen (8) eine Blasform (100) aufweist und die Vorrichtung (1) einen Reinraum (6) aufweist, innerhalb dessen die Kunststoffvorformlinge (10) transportiert werden können, und wobei jede Blasform (100) zwei Seitenteile (102, 104) und ein Bodenteil (106) aufweist, wobei die Seitenteile (102, 104) und das Bodenteil (106) gemeinsam einen Hohlraum (105) ausbilden, innerhalb dessen ein Kunststoffvorformling (10) zu einem Kunststoffbehältnis (20) umformbar ist und wenigstens die diesen Hohlraum (105) begrenzenden Bereiche der Seitenteile (102, 104) und des Bodenteils (106) stets innerhalb des Reinraums (6) angeordnet sind wobei wenigstens eine erste Antriebseinrichtung (112, 116) zum Bewegen der Seitenteile (102, 104) oder zum Bewegen des Bodenteils (106) vorgesehen ist, wobei die Antriebseinrichtung (112, 116) wenigstens teilweise außerhalb des Reinraums (6) angeordnet ist und eine Dichtungseinrichtung (120) vorgesehen ist, welche den Reinraum (6) gegenüber einem Bereich, in dem die Antriebseinrichtung (112, 116) angeordnet ist, abdichtet,
wobei wenigstens eine der außerhalb des Reinraums (6) angeordneten Antriebseinrichtungen (112, 116) über eine Kopplungseinrichtung (136) mit wenigstens einem Seitenteil (102, 104) gekoppelt ist und diese Kopplungseinrichtung (136) sich durch zumindest eine den Reinraum (6) begrenzende Wandung (13, 17) hindurch erstreckt und wobei eine Lagerungseinrichtung (140 a, b) zum Lagern der Kopplungseinrichtung (136) vorgesehen ist,
**dadurch gekennzeichnet, dass**
die Lagerungseinrichtung (140 a,b) an der Wandung (13, 17) befestigt ist und sich von der Befestigung nur zu einer Seite der Wandung (13, 17) erstreckt und wobei zwei Lagerungseinrichtungen (140 a, b) zum Lagern der Kopplungseinrichtung (136) vorhanden sind, wobei sich beide Lagerungseinrichtungen (140 a, b) von einer Wandung (13, 17) in Richtung Reinraum (6) erstrecken und wobei eine erste Lagerungseinrichtung (140 a, b) an der oberen Wandung (17), einem Deckel (17) des Reinraums (6) angebracht ist und eine zweite Lagerungseinrichtung (140 a, b) an der unteren Wandung (13), einem Boden (13) des Reinraums (6) angebracht ist.

2. Vorrichtung (1) nach Anspruch 1,
**dadurch gekennzeichnet, dass** die Lagerungseinrichtung (140 a,b) vollständig auf Höhe der Wandung (13, 17) beginnt und im Wesentlichen senkrecht zur Wandung (13, 17) angebracht ist.

3. Vorrichtung (1) nach Anspruch 1 oder 2,
**dadurch gekennzeichnet, dass** die Lagerungseinrichtung (140 a,b) unbeweglich mit der Wandung (13, 17) verbunden ist

4. Vorrichtung (1) nach wenigstens einem der vorangegangenen Ansprüche,
**dadurch gekennzeichnet, dass** zwei Lagerungseinrichtungen (140a, b) zum Lagern der Kopplungseinrichtung (136) vorhanden sind, wobei sich beide Lagerungseinrichtungen (140 a,b) von der Wandung (13, 17) in Richtung Reinraum (6) oder in Richtung Umgebung (12) erstrecken oder wobei sich die erste Lagerungseinrichtung (140 a,b) von der Wandung (13, 17) in Richtung Reinraum und die zweite Lagerungseinrichtung (140 a,b) in Richtung Umgebung (12) erstreckt.

5. Vorrichtung (1) nach wenigstens einem der vorangegangenen Ansprüche,
**dadurch gekennzeichnet, dass** die erste Lagerungseinrichtung (140 a,b) mit der Wandung (13,17) verschraubt ist und die zweite Lagerungseinrichtung (140 a,b) mit der Wandung (13, 17) verschweißt ist,

6. Vorrichtung (1) nach Anspruch 4,
**dadurch gekennzeichnet, dass** die erste und die zweite Lagerungseinrichtung (140 a,b) zur Lagerung einer gemeinsamen Kopplungseinrichtung (136) dienen.

7. Vorrichtung nach wenigstens einem der vorangegangenen Ansprüche,
**dadurch gekennzeichnet, dass** eine Fläche (146) der Lagerungseinrichtung (140 a) in einem Winkel zur Horizontalen ausgebildet ist, bevorzugt in einem Winkel von 5°-85°, besonders bevorzugt 10°-30°.

8. Vorrichtung (1) nach wenigstens einem der vorangegangenen Ansprüche,
**dadurch gekennzeichnet, dass** sich die Lagerungseinrichtung (140 a,b) zum Lagern der Kopplungseinrichtung (136) von der Wandung (13, 17) in Richtung Reinraum (6) erstreckt und wobei eine Abstützeinrichtung (150) vorgesehen ist, die sich auf dem Außenbereich (14 a,b) dieser Wandung (13, 17) befindet, wobei bevorzugt die Abstützeinrichtung (150) an eine Ausnehmung der Wandung (13, 17) zum Einsetzen bzw. Austauschen der gesamten Blasstation (8) angepasst ist.

9. Vorrichtung (1) nach wenigstens einem der vorangegangenen Ansprüche,
**dadurch gekennzeichnet, dass** zwischen der Kopplüngseinrichtung (136) und der Lagerungseinrichtung (140) eine Dichtungseinrichtung (144) vorgesehen ist.

10. Vorrichtung (1) nach wenigstens einem der vorangegangenen Ansprüche,
**dadurch gekennzeichnet, dass** die Kopplungseinrichtung (136) zur Betätigung einer Beaufschlagungseinrichtung (200), des Bodenteils (106) oder einer Verriegelung ausgebildet ist.

11. Vorrichtung (1) nach wenigstens einem der vorangegangenen Ansprüche,
**dadurch gekennzeichnet, dass**
die Lagerungseinrichtung (140 a,b) in einem horizontalen Abstand von höchstens 10 cm, bevorzugt höchstens 5 cm, besonders bevorzugt höchstens 3 cm, von der Blasform (100) entfernt ist.

12. Vorrichtung (1) nach wenigstens einem der vorangegangenen Ansprüche 1 bis 11,
**dadurch gekennzeichnet, dass**
sich die Lagerungseinrichtung (140 a,b) in Richtung einer Achse (X) zumindest teilweise auf Höhe der Blasform (100) befindet.

13. Anlage (50) zum Herstellen von Kunststoffbehältnissen (20) mit einer Vorrichtung (1) nach wenigstens einem der vorangegangenen Ansprüche und mit einer Heizeinrichtung (30), wobei diese Heizeinrichtung (30), in einer Transportrichtung (34) der Kunststoffvorformlinge stromaufwärts bezüglich der Vorrichtung (1) angeordnet ist.

14. Anlage (50) nach Anspruch 13,
**dadurch gekennzeichnet, dass**
die Anlage eine Sterilisationseinrichtung (32) aufweist, welche wenigstens einen Bereich der Kunststoffvorformlinge (10) vor Erreichen der Vorrichtung (1) sterilisiert.

15. Anlage (50) nach wenigstens einem der vorangegangenen Ansprüche,
**dadurch gekennzeichnet, dass**
die Anlage (50) einen weiteren Reinraum aufweist, der in der Transportrichtung der Kunststoffvorformlinge vor der Vorrichtung (1) angeordnet ist.

## Claims

1. A device (1) for reforming plastic pre-forms (10) into plastic containers (20) with a transport device (2) on which are arranged a plurality of blow moulding stations (8), wherein each of these blow moulding stations (8) has a blow mould (100) and the device (1) has a clean room (6) inside which the plastic pre-forms (10) can be transported, and wherein each blow mould (100) has two lateral parts (102, 104) and a bottom part (106), wherein the lateral parts (102, 104) and the bottom part (106) jointly form a cavity (105) inside which a plastic pre-form (10) is capable of being reformed into a plastic container (20) and at least the areas of the lateral parts (102, 104) delimiting this cavity (105) and of the bottom part (106) are always situated inside the clean room (6), wherein at least one first drive device (112, 116) is provided for moving the lateral parts (102, 104) or for moving the bottom part (106), wherein the drive device (112, 116) is situated at least in part outside the clean room (6), and a sealing device (120) is provided which seals off the clean room (6) from a region in which the drive device (112, 116) is situated, wherein at least one of the drive devices (112, 116) situated outside the clean room (6) is coupled to at least one lateral part (102, 104) by way of a coupling device (136) and this coupling device (136) extends through at least one wall (13, 17) delimiting the clean room (6) and wherein a bearing device (140 a, b) is provided for bearing the coupling device (136), **characterized in that** the bearing device (140 a, b) is fastened to the wall (13, 17) and extends from the fastening only to one side of the wall (13, 17) and wherein two bearing devices (140 a, b) are present for bearing the coupling device (136), wherein both bearing devices (140 a, b) extend from a wall (13, 17) in the direction of the clean room (6) and wherein the first bearing device (140 a, b) is arranged is arranged at the upper wall (17), i.e. a cover (17) of the clean room (6) and a second bearing device (140 a, b) is arranged at the lower wall (13), i.e. a base (13) of the clean room (6).

2. A device (1) according to claim 1, **characterized in that** the bearing device (140 a, b) starts completely at the level of the wall (13, 17) and it is attached substantially at a right angle to the wall (13, 17).

3. A device (1) according to claim 1 or 2, **characterized in that** the bearing device (140 a, b) is connected to the wall (13, 17) in an immovable manner.

4. A device (1) according to at least one of the preceding claims, **characterized in that** two bearing devices (140 a, b) for bearing the coupling device (136) are present, wherein the two bearing devices (140 a, b) extend from the wall (13, 17) in the direction of the clean room (6) or in the direction of the environment (12) or wherein the first bearing device (140 a, b) extends from the wall (13, 17) in the direction of the clean room and the second bearing device (140 a, b) extends in the direction of the environment (12).

5. A device (1) according to at least one of the preceding claims, **characterized in that** the first bearing device (140 a, b) is screwed to the wall (13, 17) and the second bearing device (140 a, b) is welded to the wall (13, 17).

6. A device (1) according to claim 4, **characterized in that** the first and the second bearing devices (140 a, b) serve for bearing a common coupling device (136).

7. A device according to at least one of the preceding claims, **characterized in that** one face (146) of the bearing device (140 a) is formed at an angle to the horizontal, preferably at an angle of from 5° to 85°, and in a particularly preferred manner at an angle of from 10° to 30°.

8. A device (1) according to at least one of the preceding claims, **characterized in that** the bearing device (140 a, b) for bearing the coupling device (136) extends from the wall (13, 17) in the direction of the clean room (6) and wherein a support device (150) is provided which is situated on the external region (14 a, b) of this wall (13, 17), wherein in a preferred manner the support device (150) is adapted to a recess in the wall (13, 17) in order to insert or replace the blow moulding station (8) as a whole respectively.

9. A device (1) according to at least one of the preceding claims, **characterized in that** a sealing device (144) is provided between the coupling device (136) and the bearing device (140).

10. A device (1) according to at least one of the preceding claims, **characterized in that** the coupling device (136) is designed to actuate a stressing device (200), the bottom part (106) or a locking means.

11. A device (1) according to at least one of the preceding claims, **characterized in that** the bearing device (140 a, b) is at a horizontal distance of at most 10 cm, preferably at most 5 cm, and in a particularly preferred manner at most 3 cm, from the blow mould (100).

12. A device (1) according to at least one of the preceding claims 1 to 11, **characterized in that** the bearing device (140 a, b) is situated at least in part at the level of the blow mould (100) in the direction of an axis (X).

13. A plant (50) for the production of plastic containers (20), with a device (1) according to at least one of the preceding claims and with a heating device (30), wherein this heating device (30) is arranged in a transport direction (34) of the plastic pre-forms upstream with respect to the device (1).

14. A plant (50) according to claim 13, **characterized in that** the plant has a sterilization device (32) which sterilizes at least one area of the plastic pre-forms (10) before reaching the device (1).

15. A plant (50) according to at least one of the preceding claims, **characterized in that** the plant (50) has a further clean room which is arranged upstream of the device (1) in the transport direction of the plastic pre-forms.

## Revendications

1. Dispositif (1) de transformation de préformes en matière synthétique (10) en récipients en matière synthétique (20) comprenant un dispositif de transport (2), sur lequel une pluralité de stations de soufflage (8) sont agencées, chacune de ces stations de soufflage (8) comprenant une moule de soufflage (100) et le dispositif (1) comprenant une salle banche (6), à l'intérieur de laquelle les préformes en matière synthétique (10) peuvent être transportées, et chaque moule de soufflage (100) comprenant deux parties latérales (102, 104) et une partie fond (106), les parties latérales (102, 104) et la partie fond (106) formant conjointement une cavité (105), à l'intérieur de laquelle une préforme en matière synthétique (10) peut être transformée en un récipient en matière synthétique (20) et au moins les zones des parties latérales (102, 104) et de la partie fond (106) délimitant cette cavité (105) étant situées en permanence à l'intérieur de la salle blanche (6), au moins un premier dispositif d'entraînement (112, 116) étant destiné à déplacer les parties latérales (102, 104) ou à déplacer la partie fond (106), le dispositif d'entraînement (112, 116) étant agencé au moins en partie à l'extérieur de la salle blanche (6) et un dispositif d'étanchéité (120) étant prévu, lequel étanchéifie la salle blanche (6) par rapport à une zone dans laquelle le dispositif d'entraînement (112, 116) est agencé,
au moins un des dispositifs d'entraînement (112, 116) agencés à l'extérieur de la salle blanche (6) étant accouplé par l'intermédiaire d'un dispositif d'accouplement (136) à au moins une partie latérale (102, 104) et ce dispositif d'accouplement (136) s'étendant à travers au moins une paroi (13, 17) délimitant la salle blanche (6) et un dispositif de support (140a, b) étant prévu pour le support du dispositif d'accouplement (136),
**caractérisé en ce que**
le dispositif de support (140a, b) est fixé sur la paroi (13, 17) et s'étend à partir de la fixation uniquement en direction d'un côté de la paroi (13, 17) et deux dispositifs de support (140a, b) destinés au support du dispositif d'accouplement (136) étant présents, les deux dispositifs de support (140a, b) s'étendant à partir d'une paroi (13, 17) en direction de la salle blanche (6) et un premier dispositif de support (140a, b) étant fixé sur la paroi supérieure (17), un couvercle (17) de la salle blanche (6) et un deuxième dispositif de support (140a, b) étant fixé sur la paroi inférieure (13), un fond (13) de la salle blanche (6).

2. Dispositif (1) selon la revendication 1,
**caractérisé en ce que** le dispositif de support (140a, b) commence dans son intégralité à hauteur de la paroi (13, 17) et est monté sensiblement perpendiculairement à la paroi (13, 17).

3. Dispositif (1) selon la revendication 1 ou 2,
**caractérisé en ce que** le dispositif de support (140a, b) est relié immobile à la paroi (13, 17).

4. Dispositif (1) selon au moins l'une des revendications précédentes,
**caractérisé en ce que** deux dispositifs de support (140a, b) destinés à soutenir le dispositif d'accouplement (136) sont présents, les deux dispositifs de support (140a, b) s'étendant à partir de la paroi (13, 17) en direction de la salle blanche (6) ou en direction de l'environnement (12) et le premier dispositif de support (140a, b) s'étendant à partir de la paroi (13, 17) en direction de la salle blanche et le deuxième dispositif de support (140a, b) s'étendant en direction de l'environnement (12).

5. Dispositif (1) selon au moins l'une des revendications précédentes,
**caractérisé en ce que** le premier dispositif de support (140a, b) est vissé à la paroi (13, 17) et le deuxième dispositif de support (140a, b) est soudé à la paroi (13, 17).

6. Dispositif (1) selon la revendication 4,
**caractérisé en ce que** le premier et le deuxième dispositifs de support (140a, b) servent au support d'un dispositif d'accouplement (136) commun.

7. Dispositif selon au moins l'une des revendications précédentes,
**caractérisé en ce qu'**une surface (146) du dispositif de support (140a) est réalisée de manière à former un angle par rapport à l'horizontale, de préférence à former un angle de 5°-85°, de manière particulièrement préférée de 10°-30°.

8. Dispositif (1) selon au moins l'une des revendications précédentes,
**caractérisé en ce que** le dispositif de support (140a, b) destiné à soutenir le dispositif d'accouplement (136) s'étend à partir de la paroi (13, 17) en direction de la salle blanche (6) et un dispositif de soutien (150) étant prévu, qui se situe sur la zone extérieure (14a, b) de cette paroi (13, 17), le dispositif de soutien (150) étant de préférence adapté à un évidement de la paroi (13, 17) pour l'insertion ou le remplacement de l'ensemble de la station de soufflage (8) respectivement.

9. Dispositif (1) selon au moins l'une des revendications précédentes,
**caractérisé en ce qu'**un dispositif d'étanchéité (144) est prévu entre le dispositif d'accouplement (136) et le dispositif de support (140).

10. Dispositif (1) selon au moins l'une des revendications précédentes,
**caractérisé en ce que** le dispositif d'accouplement (136) est conçu pour actionner un dispositif de sollicitation (200), de la partie fond (106) ou un dispositif de verrouillage.

11. Dispositif (1) selon au moins l'une des revendications précédentes,
**caractérisé en ce que**
le dispositif de support (140a, b) est espacé du moule de soufflage (100) selon un écart horizontal de maximum 10 cm, de préférence de maximum 5 cm, de manière particulièrement préférée de maximum 3 cm.

12. Dispositif (1) selon au moins l'une des revendications précédentes 1 à 11,
**caractérisé en ce que**
le dispositif de support (140a, b) se situe en direction d'un axe (X) au moins en partie à hauteur du moule de soufflage (100).

13. Installation (50) de fabrication de récipients en matière synthétique (20) comprenant un dispositif (1) selon au moins l'une des revendications précédentes et comprenant un dispositif de chauffage (30), ce dispositif de chauffage (30) étant agencé dans une direction de transport (34) des préformes en matière synthétique en amont par rapport au dispositif (1).

14. Installation (50) selon la revendication 13,
**caractérisée en ce que**
l'installation comprend un dispositif de stérilisation (32), lequel stérilise au moins une zone des préformes en matière synthétique (10) avant d'atteindre le dispositif (1).

15. Installation (50) selon au moins l'une des revendications précédentes,
**caractérisée en ce que**
l'installation (50) comprend une autre salle blanche qui est agencée devant le dispositif (1) dans la direction de transport des préformes en matière synthétique.
